(19) Europäisches Patentamt
European Patent Office
Office européen des brevets

(11) **EP 2 148 703 B1**

(12) **FASCICULE DE BREVET EUROPEEN**

(45) Date de publication et mention
de la délivrance du brevet:
**29.09.2010 Bulletin 2010/39**

(51) Int Cl.:
*A61L 15/24* (2006.01)      *A61K 31/7016* (2006.01)
*A61K 33/38* (2006.01)      *A61L 15/44* (2006.01)

(21) Numéro de dépôt: **08805837.5**

(86) Numéro de dépôt international:
**PCT/FR2008/050893**

(22) Date de dépôt: **23.05.2008**

(87) Numéro de publication internationale:
**WO 2008/149036 (11.12.2008 Gazette 2008/50)**

(54) **NOUVEL AGENT PERMETTANT LE RELARGAGE D'ACTIFS DANS DES PANSEMENTS CONTENANT AU MOINS UN CORPS GRAS**

NEUARTIGES MITTEL ZUR FREISETZUNG VON WIRKSTOFFEN IN DRESSINGS MIT MINDESTENS EINER FETTIGEN SUBSTANZ

NOVEL AGENT FOR RELEASING ACTIVE PRINCIPLES IN DRESSINGS CONTAINING AT LEAST ONE FATTY SUBSTANCE

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **25.05.2007 FR 0755272**

(43) Date de publication de la demande:
**03.02.2010 Bulletin 2010/05**

(73) Titulaire: **Laboratoires Urgo
21300 Chenove (FR)**

(72) Inventeur: **LAURENSOU, Christelle
F-21000 Dijon (FR)**

(74) Mandataire: **Hubert, Philippe et al
Cabinet Beau de Loménie
158, rue de l'Université
75340 Paris Cédex 07 (FR)**

(56) Documents cités:
**WO-A-01/70285      FR-A- 2 782 086**

• **SEPPIC: "SEPINOV TM EMT 10"[Online] avril 2005 (2005-04), pages 1-31, XP002463431 Extrait de l'Internet: URL:HTTP://WWW.IN-COSMETICSEASTERNEUROPE.C OM/ EXHIBITORLIBRARY/19/SEPINOV_EMT_2.PDF> [extrait le 2008-01-03]**

**Description**

De nombreux pansements qui utilisent des compositions constituées de corps gras, généralement à base d'huile ou de vaseline, et/ou d'une matrice élastomérique, dans lesquelles on peut incorporer des quantités plus ou moins importantes de particules d'hydrocolloïdes, sont aujourd'hui utilisés pour le traitement des plaies.

On peut ainsi citer, à titre d'exemples, les produits commercialisés sous les dénominations TULLE GRAS® par la société SOLVAY PHARMA, PHYSIOTULLE® et COMFEEL® par la société COLOPLAST, URGOTUL®, ALGOPLAQUE® et CELLOSORB® par les Laboratoires URGO.

Les compositions utilisées dans ces pansements sont généralement conçues pour ne pas adhérer à la plaie et pour favoriser la cicatrisation en créant un milieu humide au niveau de la plaie.

Pour permettre d'absorber les exsudats de la plaie, ces compositions peuvent contenir des quantités importantes d'hydrocolloïdes, comme par exemple dans les produits ALGOPLAQUE® commercialisés par les Laboratoires URGO et COMFEEL® commercialisé par Coloplast) ou être incorporé dans des pansements associés à une ou plusieurs compresses absorbantes comme par exemple dans les produits TULLE GRAS®, PHYSIOTULLE® et URGOTUL® ou complexés à une mousse absorbante comme dans le produit CELLOSORB®.

Ces compositions peuvent être également conçues pour permettre aux pansements les incorporant de tenir en place sans l'aide d'une bande adhésive complémentaire, en adhérant à la peau.

On a également conçu des compositions pour pansement incorporant diverses substances actives destinées à être relarguées afin d'agir dans le lit de la plaie ou sur ses berges dans la région généralement appelée peau périlésionnelle.

A titre d'exemples de telles substances actives, on peut ainsi citer les composés antiseptiques ou antibactériens comme les sels d'argent ; les composés qui agissent au niveau du processus de cicatrisation comme les anti-protéases, ou encore des composés agissant contre la douleur comme les anti-inflammatoires non stéroïdiens.

Des pansements réalisés à l'aide de compositions contenant une substance active sont par exemple commercialisés par la société Laboratoires URGO sous les dénominations URGOTUL® SAg ou CELLOSORB® Ag.

Toutefois, l'incorporation d'une substance active dans ces compositions à base de corps gras et/ou de matrice élastomérique est un problème complexe.

La nature hydrophobe de ces compositions rend difficile l'incorporation en leur sein de substances actives hydrophiles.

De plus, la substance active est généralement libérée au voisinage immédiat des exsudats de la plaie. De ce fait, suivant sa solubilité dans les milieux hydrophiles, la substance active a une tendance plus ou moins marquée à rester emprisonnée dans la composition et par conséquent à être disponible. Il peut donc être nécessaire d'incorporer des quantités importantes de substance active lorsque celle-ci a tendance à rester emprisonnée dans la composition.

Par ailleurs, l'addition d'une substance active dans une composition pour pansement doit être réalisée sans remettre en cause les propriétés de cohésion, d'absorption (voire d'adhésion, lorsque le pansement adhère à la peau), lesquelles sont toujours difficiles à obtenir, en particulier dans le cas de compositions qui contiennent des quantités importantes d'hydrocolloïdes.

L'addition d'une substance active dans une composition pour pansement doit encore être réalisée en évitant les éventuels problèmes d'interaction entre ladite substance et les constituants de la composition.

Afin de remédier à ces problèmes, il a été proposé dans la demande de brevet EP 272 149 d'incorporer la substance active à l'hydrocolloïde dont le gonflement par suite de l'absorption des exsudats permet le relargage.

Cette solution, outre qu'elle est complexe à mettre en oeuvre, n'est pas adaptée aux compositions contenant peu d'hydrocolloïdes et a fortiori aux compositions ne comprenant pas d'hydrocolloïdes. Pour de telles compositions, il a été proposé dans le brevet EP 1 272 229 d'incorporer à la composition un tensioactif, et en particulier le produit commercialisé sous la dénomination MONTANOX® 80, afin de favoriser le relargage de la substance active, en l'occurrence une substance antibactérienne telle qu'un sel d'argent.

La demande de brevet WO 01/70285 décrit également des compresses antiseptiques comprenant un tensioactif tel que le MONTANOX® 80 en tant qu'agent de relargage.

La présence d'un tensioactif au sein d'une composition pour pansement soulève toutefois un autre problème. En effet, en raison de leurs propriétés amphiphiles, les composés tensioactifs sont connus pour agir au niveau des parois des cellules et conduire à leur lyse.

Dans le cadre du processus de cicatrisation où l'on souhaite refermer la plaie, on cherche, en particulier lors de la phase de bourgeonnement, à induire ou accélérer la prolifération cellulaire et plus précisément la prolifération des fibroblastes qui sont les cellules incontournables de cette phase et qui vont conduire à la reconstruction du tissu conjonctif qui permet de refermer la plaie. Or, les tensioactifs et en particulier le MONTANOX® 80 décrit dans ce brevet antérieur est toxique pour les fibroblastes.

Dans ces conditions, il serait donc souhaitable de disposer d'un agent de relargage qui ne soit pas un tensioactif afin de ne pas nuire à la prolifération cellulaire au cours de ce processus de cicatrisation, voire même de favoriser cette prolifération cellulaire.

Il serait en outre souhaitable que cet agent de relargage puisse agir à des concentrations voisines de celles d'un tensioactif afin d'éviter une reformulation complète (qualitative et quantitative) de la composition.

La présente invention a pour but de résoudre le problème technique consistant en la fourniture d'un nouvel agent de relargage d'une substance active, qui ne soit pas toxique pour les cellules en particulier les fibroblastes, qui soit facilement incorporable dans les compositions pour pansement à base de corps gras et/ou de matrice élastomérique en présence ou non d'hydrocolloïdes, sans altérer les propriétés de cohésion, d'absorption ou d'adhésion desdites compositions, et ceci quelle que soit la solubilité de la substance active pour les liquides et notamment pour les exsudats de la plaie.

Il a été découvert, et ceci constitue le fondement de la présente invention, qu'il était possible de résoudre ce problème technique d'une façon particulièrement simple et utilisable à l'échelle industrielle en utilisant, en tant qu'agent de relargage d'une substance active, un copolymère d'un sel de l'acide 2-méthyl-2-[(1-oxo-2-propènyl)amino]-1-propanesulfonique et de l'ester 2-hydroxyéthyle de l'acide propénoïque.

Par sel de l'acide 2-méthyl-2-[(1-oxo-2-propènyl)amino]-1-propanesulfonique, on entend ici tout type de sels connus de l'homme du métier tels que les sels de sodium, de potassium, d'ammonium, etc. Dans la présente invention, le sel de sodium de l'acide 2-méthyl-2-[(1-oxo-2-propènyl)amino]-1-propanesulfonique est utilisé de manière préférentielle.

Le copolymère précité est un produit connu en tant que tel, notamment dans le domaine cosmétique, en raison de ses propriétés émulsionnantes-stabilisantes et de son bon pouvoir épaississant. Un tel produit est par exemple commercialisé par la société SEPPIC sous la dénomination commerciale SEPINOV EMT 10®.

Ainsi, selon un premier aspect, la présente invention a pour objet l'utilisation, en tant qu'agent de relargage d'une substance active dans une composition pour pansement, d'un copolymère d'un sel de l'acide 2-méthyl-2-[(1-oxo-2-propènyl)amino]-1-propanesulfonique et de l'ester 2-hydroxyéthyle de l'acide propénoïque.

Ce copolymère est particulièrement avantageux dans la mesure où, lorsqu il est incorporé dans des masses comprenant au moins un corps gras et/ou une matrice élastomérique, il permet de relarguer des substances actives variées dans des proportions équivalentes à celles obtenues avec un tensioactif.

Il a par ailleurs été découvert que ce copolymère, à la différence des composés tensioactifs connus, a tendance à induire la prolifération des fibroblastes, ce qui constitue un avantage particulièrement intéressant. Plus précisément, lorsque l'on étudie l'effet d'un tensioactif classique, tel qu'un polysorbate 80, sur la cicatrisation, plus précisément sur les fibroblastes, on s'aperçoit que ce dernier présente une toxicité vis à vis de ces cellules. En revanche, il a été constaté qu'un copolymère d'un sel de l'acide 2-méthyl-2-[(1-oxo-2-propènyl)amino]-1-propanesulfonique et de l'ester 2-hydroxyéthyle de l'acide propénoïque a, quant à lui, tendance à induire la prolifération des fibroblastes.

Selon un second aspect, la présente invention concerne un pansement du type comprenant au moins un corps gras et/ou une matrice élastomérique et au moins une substance active caractérisé en ce qu'il comprend un copolymère tel que défini précédemment.

Par pansement on entend couvrir ici tout type de pansements occlusifs ou non occlusifs.

Comme pansements non occlusifs on peut citer des pansements interfaces, tels que ceux commercialisés sous les dénominations commerciales Tulle Gras® (par SOLVAY PHARMA), Physiotulle® (par COLOPLAST) ou encore Urgotul® (par les Laboratoires URGO).

Ces pansements interfaces se présentent généralement sous- forme d'une trame ou d'un filet enduit d'une masse comprenant au moins un corps gras et/ou une matrice élastomérique.

Ils peuvent également être constitués d'une masse sans trame ou filet, ayant la forme d'une plaque présentant ou non des trous traversants, en fonction du type de plaie sur lequel le pansement est appliqué (on utilisera préférentiellement une plaque présentant des trous traversants sur une plaie exsudative lorsque la masse n'a qu'un faible ou aucun pouvoir absorbant, les trous permettant ainsi l'évacuation des exsudats de la plaie).

Ces pansements interfaces peuvent contenir une ou plusieurs substances actives. De tels pansements contenant des substances actives sont par exemple commercialisés sous les dénominations commerciales Urgotul® SAg (par les Laboratoires URGO) contenant de la sulfadiazine argentique, Corticotulle Lumière® (par SOLVAY PHARMA) contenant de la néomycine sulfate et de la polymyxine B sulfate.

Les pansements occlusifs sont pour la plupart constitués de plusieurs couches, avec une couche interne qui entre en contact avec la plaie et une couche externe.

Ces pansements peuvent se présenter sous la forme d'un pansement interface complexé à une compresse absorbante, ladite compresse pouvant être elle-même complexée à un support adhésivé. Ce type de pansement est connu et commercialisé par les Laboratoires Urgo sous la dénomination commerciale Cellosorb Ag® (dans ce cas, le pansement interface contient du sulfate d'argent en tant que substance active).

La présente invention trouve également application pour la réalisation de pansements à base d'hydrogels ou d'hydrocolloïdes dans lesquels est incorporé le copolymère précité.

Des pansements à base d'hydrocolloïdes connus sont par exemple commercialisés sous les dénominations Algoplaque® (par les Laboratoires Urgo), Duoderm® (par Convatec), Comfeel® (par Coloplast). De tels pansements sont décrits dans les demandes de brevet suivantes : FR 2 392 076, FR 2 495 473 et WO 98/10801, EP 264 299.

Dans le cadre de la présente invention, on préférera les pansements qui utilisent des compositions constituées de corps gras et/ou d'une matrice élastomérique et un hydrocolloïde ou des particules d'hydrocolloïde.

Par hydrocolloïde ou particules d'hydrocolloïde, on entend ici les composés utilisés par l'homme de l'art pour leur aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium et les polymères synthétiques à base de sels de l'acide acrylique, connus sous l'appellation "superabsorbants", comme par exemple les produits commercialisés par la société BASF sous la dénomination LUQUASORB® 1003 ou par la société CIBA Specialty chemicals sous la dénomination SALCARE® SC91.

Ces hydrocolloïdes pourront être utilisés seuls ou en association.

Les hydrocolloïdes préférés dans le cadre de la présente invention sont les sels de métal alcalin de carboxyméthylcellulose, en particulier la carboxyméthylcellulose de sodium.

La quantité d'hydrocolloïde incorporé dans la matrice polymère sera adaptée en fonction du niveau d'absorption recherché pour ladite masse. Ainsi, la quantité d'hydrocolloïde pourra être de l'ordre de 2 à 50% en poids, rapportée au poids total de la masse.

Dans le cadre de la présente invention, on utilisera de préférence une quantité d'hydrocolloïde comprise entre 20 et 50% en poids rapportée au poids total de la masse si l'on désire réaliser un pansement absorbant tel que ceux décrits dans FR 2 495 473, FR 2 392 076 ou WO 98/10801.

On utilisera de préférence une quantité d'hydrocolloïde comprise entre 2 et 20% en poids, rapportée au poids total de la masse si l'on souhaite réaliser un pansement peu absorbant comme celui décrit dans WO 00/16723.

Dans la présente description, on entend désigner par «corps gras » toute substance ou tout mélange de substances choisie(s) parmi les huiles, les graisses et les corps lipidiques (comprenant les acides gras, glycérols, stérols et leurs dérivés) d'origine naturelle (minérale, animale ou végétale) ou synthétique et se présentant à l'état liquide, semi-solide ou solide, ces substances pouvant être de poids moléculaire et d'architecture (monomérique ou polymérique) variées.

Parmi les huiles minérales pouvant être utilisées dans le cadre de la présente invention, on peut citer à titre d'exemples, les huiles de paraffine, la vaseline, et plus généralement les huiles minérales formées de composés de nature paraffinique, naphténique ou aromatique ou de leurs mélanges dans des proportions variables.

On peut ainsi citer, comme exemples d'huiles minérales, les produits commercialisés par la société SHELL sous la dénomination ONDINA® et RISELLA® pour les mélanges à base de composés naphténiques et paraffiniques ou sous la dénomination CATENEX® pour les mélanges à base de composés naphténiques, aromatiques et paraffiniques.

Dans le cadre de la présente invention, on préférera les huiles de paraffine et en particulier l'huile commercialisée par la société SHELL sous la dénomination ONDINA® 917.

Les huiles ou graisses végétales peuvent également être utilisées dans le cadre de la présente invention, comme en particulier les huiles d'arachide, de coco, de maïs, d'amande douce. Ces huiles ou graisses végétales peuvent être hydrogénées ou peroxydées.

Des graisses et huiles animales sont également appropriées, comme en particulier l'huile de suif, la lanoline ou l'huile de baleine.

Dans le cadre de la présente invention, on préférera les pansements qui utilisent des compositions constituées de corps gras et d'une matrice élastomérique et des hydrocolloïdes ou particules d'hydrocolloïde.

Par matrice élastomérique, on entend ici les compositions constituées à partir d'un ou plusieurs élastomères choisis parmi les copolymères séquencés poly(styrène-oléfine-styrène) et un ou plusieurs composés choisis parmi les produits dits tackifiants, les plastifiants, de préférence des plastifiants liquides. De telles compositions sont ainsi définies dans "Advances in Pressure Sensitive Adhesive Technology" édité par Donatas Satas en avril 1995 dans le chapitre 7 "Wound dressings" pages 158 à 171 et sont aussi parfaitement connues de l'homme de l'art.

Les élastomères du type copolymères séquences (styrène-oléfine-styrène) qui sont susceptibles d'être utilisés dans le cadre de la présente invention sont ceux habituellement utilisés par l'homme de l'art dans la préparation des pansements. Ils peuvent être, si nécessaire, associés à des copolymères séquencés (styrène-oléfine).

Ces polymères séquencés sont donc soit des copolymères triblocs du type ABA comportant deux blocs terminaux thermoplastiques A styrène et une séquence centrale élastomère B qui est une oléfine, soit des copolymères diblocs du type AB comportant un bloc thermoplastique A styrène et une séquence élastomère B qui est une oléfine. Les séquences B oléfines de ces copolymères peuvent être constituées d'oléfines insaturées comme par exemple isoprène ou butadiène ou d'oléfines saturées comme par exemple éthylène-butylène ou éthylène- propylène.

Dans le cas d'un mélange de copolymères triblocs ABA et de copolymères diblocs AB, on pourra employer des mélanges de copolymères triblocs ABA et de copolymères diblocs AB commerciaux déjà disponibles ou réaliser un mélange en toute proportion préalablement choisie à partir de deux produits disponibles indépendamment.

Les produits à séquence centrale insaturée sont bien connus de l'homme de l'art et sont par exemple commercialisés par la société KRATON POLYMERS sous la dénomination KRATON® D. On peut aussi citer pour les copolymères poly(styrène-isoprène-styrène) (en abrégé SIS) les produits commercialisés sous les dénominations KRATON® D1107 ou KRATON® D1119 BT et pour les copolymères poly(styrène-butadiène-styrène) le produit commercialisé sous la dénomination KRATON® D1102. D'autres copolymères poly(styrène-isoprène-styrène) sont aussi commercialisés par la société EXXON MOBIL CHEMICAL sous la dénomination VECTOR® comme par exemple le produit commercialisé sous la dénomination VECTOR® 4113.

Comme exemples de mélanges commerciaux de copolymères triblocs ABA et diblocs AB dans lesquels B est de l'isoprène, on peut citer le produit commercialisé par la société EXXON MOBIL CHEMICAL sous la dénomination VECTOR® 4114 ou le produit VECTOR® désigné par le code DPX-565.

Tous ces copolymères à base d'isoprène ou de butadiène présentent généralement une teneur en styrène comprise entre 10 et 52% en poids rapportée au poids total dudit copolymère.

Dans le cadre de la présente invention, on pourra utiliser les copolymères séquencés triblocs poly(styrène-isoprène-styrène) (en abrégé SIS) ayant une teneur en styrène comprise entre 14 et 30% en poids par rapport au poids dudit SIS.

D'une façon particulièrement préférée, on utilisera le produit commercialisé par la société KRATON POLYMERS sous la dénomination KRATON® D1111K comme copolymère séquence triblocs poly(styrène-isoprène-styrène) et le produit VECTOR® DPX-565 commercialisé par la société EXXON MOBIL CHEMICAL comme mélange de copolymère séquencé triblocs poly(styrène-isoprène-styrène) et de copolymère séquence diblocs poly(styrène-isoprène).

Les produits à séquence centrale saturée sont aussi bien connus de l'homme de l'art et sont par exemple commercialisés par la société KRATON POLYMERS sous la dénomination KRATON® G pour les copolymères séquencés poly(styrène-éthylène-butylène-styrène) (en abrégé SEBS) comme en particulier les produits KRATON® G1651, KRATON® G1654 ou KRATON® G1652 ou par la société KURARAY sous la dénomination SEPTON® pour les copolymères séquencés poly(styrène-éthylène-propylène-styrène) (en abrégé SEPS).

Comme exemple de mélanges commerciaux de copolymères tribloc-dibloc, on peut citer le produit commercialisé par la société KRATON POLYMERS sous la dénomination KRATON® G1657 dont la séquence oléfine est éthylène-butylène.

Comme exemple d'un mélange particulier tribloc-dibloc que l'on peut utiliser dans le cadre de la présente invention, on peut citer le mélange d'un SEBS tribloc comme le produit commercialisé par la société KRATON POLYMERS sous la dénomination KRATON® G1651 avec un matériau dibloc poly(styrène-oléfine) comme le poly(styrène-éthylène-propylène) commercialisé par la société KRATON POLYMERS sous la dénomination KRATON® G1702.

Dans le cadre de la présente invention, on préférera les copolymères triblocs SEBS ou SEPS ayant une teneur en styrène comprise entre 25 et 45% en poids par rapport au poids dudit SEBS. On préférera tout particulièrement les produits commercialisés par la société KRATON POLYMERS sous les dénominations KRATON® G1651 et KRATON® G1654.

De façon générale, l'élastomère thermoplastique sera utilisé, selon la nature du copolymère séquencé, en une quantité de l'ordre de 2 à 40% en poids, rapportée au poids total de la composition.

Si nécessaire, on pourra ajouter à ces copolymères séquencés des agents antioxydants. Par agents antioxydants, on entend désigner ici les composés couramment employés par l'homme de l'art pour assurer la stabilité vis-à-vis de l'oxygène, la chaleur, l'ozone et les rayonnements ultra violets des composés utilisés dans la formulation des masses hydrocolloïdes, en particulier les résines tackifiantes et les copolymères séquencés. On peut utiliser un ou plusieurs de ces agents antioxydants en association.

Comme exemples d'agents antioxydants appropriés, on peut citer les antioxydants phénoliques comme en particulier les produits commercialisés par la société CIBA SPECIALTY CHEMICALS sous les dénominations IRGANOX® 1010, IRGANOX® 565, IRGANOX® 1076 et des antioxydants soufrés comme en particulier le dibutyldithiocarbamate de zinc commercialisé par la société AKZO sous la dénomination PERKACIT ZDBC.

Ces antioxydants pourront être utilisés en une quantité de l'ordre de 0,05 à 1% en poids par rapport au poids total de la masse hydrocolloïde.

Dans le cadre de la présente invention, on préférera l'utilisation de l'IRGANOX® 1010.

Pour obtenir des masses élastomériques adhésives, on peut également ajouter à ces copolymères séquencés des produits dits "tackifiants" tels que ceux qui sont habituellement utilisés par l'homme de l'art dans la préparation des adhésifs sensibles à la pression comprenant des élastomères et en particulier des copolymères séquences poly(styrène-oléfine-styrène) et l'on pourra se reporter à cet égard au document de l'état de la technique mentionné précédemment ou à l'ouvrage de Donatas Satas "Handbook of Pressure Sensitive Technology".

Dans le cadre de la présente invention, on pourra donc utiliser un ou des produits tackifiants dans une large proportion de l'ordre de 1 à 70% en poids rapportée au poids total de la composition, en fonction des autres éléments de cette dernière, pour obtenir le pouvoir adhésif souhaité pour la composition finale.

De préférence, on utilisera un produit tackifiant ou un ensemble de produits tackifiants dans une proportion de 10 à 40% en poids, rapportée au poids total de la composition.

D'une façon générale, ces produits tackifiants sont choisis parmi les résines tackifiantes, les polyisobutylènes de bas poids moléculaire et les polybutènes de bas poids moléculaire ou leurs mélanges.

Parmi les résines tackifiantes qui conviennent selon l'invention, on peut mentionner les résines polyterpènes ou terpènes modifiées, les résines de colophane, les résines hydrocarbonnées, les mélanges de résine cyclique, aromatique et aliphatique, etc., ou des mélanges de ces résines.

De tels produits sont par exemple commercialisés par la société GOODYEAR sous la dénomination WINGTACK® comme en particulier la résine de synthèse formée de copolymères en $C_5/C_9$ commercialisée sous la dénomination WINGTACK® 86 ou la résine à base de polyterpène synthétique commercialisée sous la dénomination WINGTACK® 10. On peut aussi citer à titre d'exemple les résines commercialisées sous la dénomination KRISTALEX® par la société HERCULES comme en particulier la résine à base d'alpha-méthylstyrène KRISTALEX® 3085.

Dans le cadre de la présente invention, on préférera les résines commercialisées par la société EXXON MOBIL CHEMICAL sous la dénomination ESCOREZ® et tout particulièrement la résine de synthèse commercialisée sous la dénomination ESCOREZ® 5380.

Comme polybutènes de bas poids moléculaire utilisables en tant que produit tackifiant de la matrice élastomérique, on peut citer les produits bien connus de l'homme de l'art qui sont par exemple commercialisés sous la dénomination NAPVIS® par la société BP CHIMIE.

Dans le cadre de la présente invention, on préférera tout particulièrement le produit commercialisé sous la dénomination NAPVIS® 10.

Ces polybutènes peuvent être utilisés seuls ou en mélange.

Ils seront utilisés de préférence dans une proportion de 5 à 30% en poids par rapport au poids total de la composition et plus particulièrement de 8 à 15% en poids.

Dans le cadre de la présente invention, on entend par "plastifiant " les plastifiants qui sont habituellement utilisés par l'homme de l'art pour la préparation des adhésifs sensibles à la pression comprenant des élastomères thermoplastiques en particulier du type copolymères séquencés poly(styrène-oléfine-styrène) et qui sont des produits qui permettent d'améliorer leurs propriétés d'étirement, de souplesse, d'extrudabilité ou de mise en oeuvre et l'on pourra se reporter à cet égard aux documents de l'état de la technique mentionnés précédemment.

Ces plastifiants, qui sont de préférence des plastifiants liquides, sont des composés compatibles avec la séquence centrale oléfine des copolymères séquencés utilisés. Comme plastifiant liquide, on peut utiliser des huiles plastifiantes, et en particulier des huiles minérales qui sont formées de composés de nature paraffinique, naphténique ou aromatique ou de leurs mélanges dans des proportions variables.

On peut ainsi citer, à titre d'exemples d'huiles minérales, les produits commercialisés par la société SHELL sous la dénomination ONDINA® et RISELLA® pour les mélanges à base de composés naphténiques et paraffiniques ou sous la dénomination CATENEX® pour les mélanges à base de composés naphténiques, aromatiques et paraffiniques.

Dans le cadre de la présente invention, on utilisera de préférence les huiles de paraffine et en particulier l'huile commercialisée par la société SHELL sous la dénomination ONDINA® 917.

On peut aussi utiliser comme plastifiant liquide non pas une huile plastifiante mais des produits de synthèse à base de mélanges liquides d'hydrocarbures saturés comme par exemple les produits commercialisés par la société TOTAL sous la dénomination GEMSEAL® comme en particulier le produit GEMSEAL® 60 qui est un mélange isoparaffinique issu d'une coupe pétrolière totalement hydrogénée.

Dans le cadre de la réalisation d'une masse hydrocolloïde selon l'invention, on utilisera de préférence un plastifiant liquide en une concentration de l'ordre de 10 à 95% en poids par rapport au poids total de la composition et de préférence encore de 30 à 75% en poids par rapport au poids total de la masse hydrocolloïde.

Dans le cadre de la présente description, on entend par substance active, toute substance exerçant une activité pharmacologique comme en particulier des agents bactéricides ou bactériostatiques (chloramine, chlorhexidine, sels d'argent, de zinc, metronidazole, pénicilline...), des agents favorisant la cicatrisation (hormones, peptides...), des enzymes favorisant la détersion de la plaie (pepsine, trypsine...), des inhibiteurs de protéase ou métalloprotéase, des agents anti-douleurs ou des anesthésiques locaux (lidocaïne, chinchocaïne) ou des agents anti-inflammatoires non stéroïdiens (ibuprofène, kétoprofène, fénoprofène, diclofénac).

Ces substances actives peuvent être présentes dans le pansement en des concentrations allant de 0,01 à 15% en poids, de préférence de 3 à 8% en poids, ces concentrations pouvant varier en fonction de la substance active utilisée.

Les propriétés de relargage du copolymère utilisé dans le cadre de la présente invention ainsi que ses propriétés sur les fibroblastes ont été mises en évidence dans les exemples donnés ci-après. Il a été constaté que ces propriétés s'exercent à faible dose, c'est-à-dire lorsque le pansement comprend de 0,1 à 20% en poids, de préférence de 1 à 10% en poids et de préférence encore environ 5% en poids de copolymère d'un sel de l'acide 2-méthyl-2-[(1-oxo-2-propényl) amino]-1-propane-sulfonique et de l'ester 2-hydroxyéthyle de l'acide propénoïque.

**Mise en évidence des propriétés du copolymère utilisé dans le cadre de l'invention**

a. Composants utilisés

Les différents composants suivants ont été utilisés afin de réaliser les différents pansements de la présente invention sont les :

SEPINOV EMT 10 : un copolymère d'un sel de l'acide 2-méthyl-2-[(1-oxo-2-propènyl)amino]-1-propanesulfonique et de l'ester 2-hydroxyéthyle de l'acide propénoïque, commercialisé par la société SEPPIC
Kraton G 1654 et G 1651 : Styrène Ethylène Butylène Styrène (S-EB-S) à haut poids moléculaire commercialisé par la société KRATON
Kraton D1111K : Copolymère de Styrène-Isoprène-Styrène (SIS) contenant au moins 22% de polystyrène commercialisé par la société KRATON
Ondina 917 : huile minérale commercialisée par la société SHELL
Irganox 1010: Pentaerythritol Tetrakis (3-(3,5-di-tert-butyl-4-hydroxy-phenyl)propionate) commercialisé par la sociétés CIBA SPECIALTY CHEMICALS
Vaseline Codex A : vaseline commercialisée par la société AIGLON
CMC Blanose 7H4XF : carboxyméthylcellulose sodique commercialisée par la société HERCULES
Luquasorb 1003 : polymère superabsorbant de polyacrylate de sodium commercialisé par BASF.
Sulfadiazine argentique commercialisée par la société ARGENOL BENTLEY Sucrose octasulfate de potassium commercialisé par la société EUTICALS.

b$_1$. Fabrication de masses élastomériques

Exemples 1 à 8

Les masses élastomériques des exemples 1 à 8 ont été préparées par malaxage dans un malaxeur à bras en Z. La température de consigne des exemples 3 et 4 était de 140°C, celle des exemples 1, 2, 5 à 8 de 105°C :

1. Les élastomères triblocs styrène-éthylènebutylène-styrène ou styrène-isoprène-styrène ont été mélangés à la moitié de l'huile minérale et à l'antioxydant.
2. A la 30$^{ème}$ minute, la vaseline a été ajoutée au mélange.
3. A la 40$^{ème}$ minute, le reste d'huile minérale a été ajoutée.
4. A la 55$^{ème}$ minute, la carboxyméthylcellulose sodique ou le polymère superabsorbant selon le cas, la substance active, éventuellement la résine tackifiante et, le cas échéant, le copolymère SEPINOV EMT 10 ont été ajoutés.

La vidange du malaxeur a été effectuée à la 70$^{ème}$ minute.

Exemple 9

La masse élastomérique de l'exemple 9 contenant le copolymère SEPINOV EMT 10 et du sucrose octasulfate de potassium (commercialisé par la société EUTICALS) en tant que substance active a été préparée par malaxage dans un malaxeur à bras en Z à une température de consigne de 110°C selon le procédé suivant :

1. La vaseline (Vaseline Codex A commercialisée par la société AIGLON) et l'huile minérale (Ondina 917 commercialisée par la société SHELL) ont été mélangées à une température de 89°C.
2. A la 7$^{ème}$ minute, le copolymère a été ajouté.
3. A la 12$^{ème}$ minute, la carboxyméthylcellulose sodique (CMC Blanose 7H4XF commercialisé par la société HERCULES) et la substance active ont été ajoutés.
4. A la 20$^{ème}$ minute, le S-EB-S à haut poids moléculaire (Kraton G 1654 commercialisé par la société KRATON) et l'antioxydant (IRGANOX 1010 commercialisé par la société CIBA SPECIALTY CHEMICALS) ont été ajoutés. La température du mélange était alors de 106°C.
5. A la 60$^{ème}$ minute, la résine tackifiante (Escorez 5380 commercialisé par la société EXXON MOBIL CHEMICAL) a été ajoutée.

La vidange du malaxeur a été effectuée à la 80$^{ème}$ minute.

Exemple 10

La masse élastomérique de l'exemple 10 contenant du sucrose octasulfate de potassium (commercialisé par la société EUTICALS) en tant que substance active a été préparée par malaxage dans un malaxeur à bras en Z à une température de consigne de 110°C selon le procédé suivant :

1. La vaseline (Vaseline Codex A commercialisée par la société AIGLON) et l'huile minérale (Ondina 917 commercialisée par la société SHELL) ont été mélangées à une température de 89°C.
2. A la 3ème minute, la carboxyméthylcellulose sodique (CMC Blanose 7H4XF commercialisé par la société HERCULES) et la substance active ont été ajoutés.
3. A la 10ème minute, le S-EB-S à haut poids moléculaire (Kraton G 1654 commercialisé par la société KRATON) et l'antioxydant (IRGANOX 1010 commercialisé par la société CIBA SPECIALTY CHEMICALS) ont été ajoutés. La température du mélange était alors de 104°C.
4. A la 40ème minute, la résine tackifiante (Escorez 5380 commercialisé par la société EXXON MOBIL CHEMICAL) a été ajoutée.

La vidange du malaxeur a été effectuée à la 55ème minute.

Exemple 11

La masse élastomérique de l'exemple 11 contenant le copolymère SEPINOV EMT 10 et de la sulfadiazine argentique (commercialisée par la société ARGENOL BENTLEY) en tant que substance active a été préparée par malaxage dans un malaxeur à bras en Z à une température de consigne de 130°C selon le procédé suivant :

1. Le copolymère Sepinov EMT 10 et l'huile minérale (Ondina 917 commercialisée par la société SHELL) ont été mélangés à une température de 110°C.
2. A la 5ème minute, la carboxyméthylcellulose sodique (CMC Blanose 7H4XF commercialisé par la société HERCULES), la substance active et la vaseline (Vaseline Codex A commercialisée par la société AIGLON) ont été ajoutés.
3. A la 12ème minute, le S-EB-S à haut poids moléculaire (Kraton G 1651 commercialisé par la société KRATON) et l'antioxydant (IRGANOX 1010 commercialisé par la société CIBA SPECIALTY CHEMICALS) ont été ajoutés. La température du mélange était alors de 106°C.
4. A la 55ème minute, la résine tackifiante (Escorez 5380 commercialisé par la société EXXON MOBIL CHEMICAL) a été ajoutée.

La vidange du malaxeur a été effectuée à la 70ème minute.

Exemple 12

La masse élastomérique de l'exemple 12 contenant la sulfadiazine argentique (commercialisée par la société ARGENOL BENTLEY) en tant que substance a été préparée par malaxage dans un malaxeur à bras en Z à une température de consigne de 130°C selon le procédé suivant :

1. L'huile minérale (Ondina 917 commercialisée par la société SHELL), la carboxyméthylcellulose sodique (CMC Blanose 7H4XF commercialisé par la société HERCULES) et la substance active ont été mélangés à une température de 92°C.
2. A la 2ème minute, la vaseline (Vaseline codex A commercialisée par la société AIGLON) a été ajoutée.
3. A la 7ème minute, le S-EB-S à haut poids moléculaire (Kraton G 1651 commercialisé par la société KRATON) et l'antioxydant (IRGANOX 1010 commercialisé par la société CIBA SPECIALTY CHEMICALS) ont été ajoutés. La température du mélange était alors de 113°C.
4. A la 41ème minute, la résine tackifiante (Escorez 5380 commercialisé par la société EXXON MOBIL CHEMICAL) a été ajoutée.

La vidange du malaxeur a été effectuée à la 60ème minute.
Les quantités (exprimées en poids pour 100 grammes) des différents constituants des masses élastomériques ainsi réalisées sont indiquées dans le tableau 1.

b$_2$. Fabrication de masses à base de vaseline

Exemples 13 à 18

Les masses à base de vaseline des exemples 13 à 18 ont été préparées à 45°C dans un bêcher selon le procédé suivant :

La vaseline (Vaseline Codex A commercialisée par la société AIGLON), la substance active (sucrose octasulfate de potassium commercialisé par la société EUTICALS ou sulfadiazine argentique de la société ARGENOL BENTLEY), le cas échéant, la carboxyméthylcellulose (CMC Blanose 7H4XF de la société HERCULES) et, le cas échéant, le copolymère (Sepinov EMT 10 commercialisé par la société SEPPIC) ont été mélangés manuellement.

Les quantités (exprimées en poids pour 100 grammes) des différents constituants de ces masses sont indiquées dans le tableau 2.

Tableau 1

| | Ondina 917 | Kraton G 1651 | Kraton G 1654 | Kraton D 1111K | Irganox 1010 | Vaseline Codex A | Blanose 7H4XF | CMC Luquasorb Escorez 1003 | Escorez 5380 | octasulfate Sucrose octasulfate de potassium | Sulfadiazine argentique | Sepinov EMT10 |
|---|---|---|---|---|---|---|---|---|---|---|---|---|
| Ex.1 | 62,38 | | 6 | | 0,12 | 5 | 14 | | | 7,5 | | 5 |
| Ex. 2 | 67,38 | | 6 | | 0,12 | 5 | 14 | | | 7,5 | | |
| Ex. 3 | 71,2 | 4,93 | | | 0,12 | 5 | 10 | | | | 3,75 | 5 |
| Ex. 4 | 76,2 | 4,93 | | | 0,12 | 5 | 10 | | | | 3,75 | |
| Ex. 5 | 62,4 | | 6 | | 0,1 | 5 | | 14 | | 7,5 | | 5 |
| Ex. 6 | 67,4 | | 6 | | 0,1 | 5 | | 14 | | 7,5 | | |
| Ex. 7 | 38,4 | | | 10 | 0,1 | 5 | | 14 | 20 | 7,5 | | 5 |
| Ex.8 | 43,4 | | | 10 | 0,1 | 5 | | 14 | 20 | 7,5 | | |
| Ex. 9 | 27,38 | | 6 | | 0,12 | 5 | 14 | | 35 | 7,5 | | 5 |
| Ex. 10 | 32,38 | | 6 | | 0,12 | 5 | 14 | | 35 | 7,5 | | |
| Ex. 11 | 36,2 | 4,93 | | | 0,12 | 5 | 10 | | 35 | | 3,75 | 5 |
| Ex. 12 | 41,2 | 4,93 | | | 0,12 | 5 | 10 | | 35 | | 3,75 | |

Tableau 2

|  | Vaseline Codex A | CMC Blanose 7H4XF | sucrose octasulfate de potassium | Sulfadiazine argentique | Sepinov EMT 10 |
|---|---|---|---|---|---|
| Exemple 13 | 78 | 9,5 | 7,5 |  | 5 |
| Exemple 14 | 83 | 9,5 | 7,5 |  |  |
| Exemple 15 | 82,5 |  | 7,5 |  | 10 |
| Exemple 16 | 92,5 |  | 7,5 |  |  |
| Exemple 17 | 86,25 |  |  | 3,75 | 10 |
| Exemple 18 | 96,25 |  |  | 3,75 |  |

c. Fabrication des pansements et produits testés

$C_1$. Des pansements interfaces constitués d'une trame enrobée d'une masse élastomérique ont été élaborés à l'aide des masses élastomériques précitées des exemples 1 à 4.

Plus précisément, on a utilisé ici une trame formée d'une marquisette thermofixée en fils de polyester (polyéthylène téréphtalate), 33 décitex en chaîne et en trame, présentant des mailles carrées dont l'ouverture est d'environ 0.8 à 1 mm$^2$ (trame 555 commercialisée par la société MDB TEXINOV).

Cette trame a été enrobée d'une couche de masse élastomérique fondue à 135-145°C, puis l'excédent a été éliminé par passage entre deux cylindres fixes dont l'écart est de 200 $\mu$m. La bande ainsi obtenue a été découpée puis complexée avec un film protecteur en polyester d'épaisseur de 23 $\mu$m sur chacune de ses faces formant ainsi des pansements individuels conditionnés dans des pochettes étanches et stérilisés sous rayonnement $\beta$ à 25 kGy.

$c_2$. Des plaques de masses élastomériques des exemples 5 à 12 ont été réalisées entre 2 feuilles de polyester siliconé de 75 $\mu$m, au moyen d'une presse chauffante dont les deux plateaux étaient réglés à 95°C. L'épaisseur de ces plaques de masse a été calibrée à l'aide de cales de 1150 $\mu$m pour obtenir des plaques de 1 mm en moyenne.

$c_3$. Les masses à base de vaseline des exemples 13 à 18 ont été pour partie enduites sur une trame viscose 552 (commercialisée par la société MDB TEXINOV) pour partie conservées afin d'analyser le relargage de la substance active contenue dans cette masse.

$c_4$. Les pansements interfaces préparés à l'aide des masses élastomériques des exemples 1 à 4 ont été complexés à chaud à une mousse polyuréthane hydrophile de 4,5 mm d'épaisseur commercialisée par la société Corpura B.V sous la dénomination commerciale VIVO MFC.03. Le complexe a été disposé entre deux plaques chauffantes sous pression à une température d'environ 100°C. Une ailette en polyester ayant une épaisseur de 50 $\mu$m a été appliquée sur le côté du pansement interface. Les pansements complexes ainsi obtenus ont été conditionnés individuellement dans des pochettes étanches et stérilisés sous rayonnement $\beta$ à 25 kGy.

Les pansements ainsi obtenus sont désignés ci-après par "exemples 19 à 22" et sont représentés au tableau 3.

Tableau 3

| Exemple 19 | Pansement de l'exemple 1+mousse polyuréthane |
|---|---|
| Exemple 20 | Pansement de l'exemple 2+mousse polyuréthane |
| Exemple 21 | Pansement de l'exemple 3+mousse polyuréthane |
| Exemple 22 | Pansement de l'exemple 4+mousse polyuréthane |

**Méthode de mesure de relargage d'une substance active**

La capacité à favoriser le relargage d'une substance active d'un copolymère d'un sel de l'acide 2-méthyl-2-[(1-oxo-2-propènyl)amino]-1-propanesulfonique et de l'ester 2-hydroxyéthyle de l'acide propénoïque est illustrée à l'aide des méthodes d'analyse suivantes :

Dans le cas des pansements interfaces, réalisés à partir des masses des exemples 1 à 4 et 13 à 18, des échantillons de 25 cm$^2$ de pansement (découpés à l'emporte pièce calibré et exactement pesés) ont été introduits dans un erlenmeyer contenant 10 ml de sérum physiologique. La verrerie, fermée hermétiquement a été placée dans une étuve à 37°C pendant 24h. Le surnageant a été prélevé et filtré. La quantité de substance active a été dosée par HPLC (Chromatographie Liquide à Haute Performance) selon les méthodes décrites ci-dessous.

<u>Dans le cas des pansements interfaces complexés à une mousse,</u> des exemples 19 à 22, le liquide a été totalement absorbé par le dispositif. Dans ce cas, le pansement a été saturé avec du sérum physiologique afin d'obtenir environ 10 ml de surnageant.

Afin de standardiser la méthode, des échantillons de 25 cm$^2$ de pansement absorbant (découpés à l'emporte pièce calibré et exactement pesés) ont été introduits dans un erlenmeyer contenant 25 ml de sérum physiologique. La verrerie, fermée hermétiquement a été placée dans une étuve à 37°C pendant 24h. Le surnageant a été prélevé et la quantité de substance active a été dosée par HPLC (Chromatographie Liquide à Haute Performance) selon les méthodes décrites ci-dessous.

<u>Dans le cas des masses sous forme de plaques,</u> des exemples 5 à 12, des échantillons de 25 cm$^2$ de plaque (découpés à l'emporte pièce calibré et exactement pesés) ont été introduits dans un erlenmeyer contenant 10 ml de sérum physiologique. La verrerie, fermée hermétiquement a été placée dans une étuve à 37°C pendant 24h. Le surnageant a été prélevé et filtré. La quantité de substance active a été dosée par HPLC (Chromatographie Liquide à Haute Performance) selon les méthodes décrites ci-dessous.

<u>Dans le cas des masses sous forme de pâte/pommade,</u> obtenues à l'aide des masses des exemples 13 à 18, 1000 mg de masse ont été étalés au fond d'un erlenmeyer contenant 10 ml de sérum physiologique. La verrerie, fermée hermétiquement a été placée dans une étuve à 37°C pendant 24h. Le surnageant a été prélevé et filtré. La quantité de substance active a été dosée par HPLC (Chromatographie Liquide à Haute Performance) selon les méthodes décrites ci-dessous.

### Méthode de dosage du sucrose octasulfate de potassium

Les conditions suivantes de dosage par Chromatographie Liquide Haute Performance (HPLC) ont été utilisées :

Réactifs

- Sulfate d'ammonium par exemple NORMAPUR code 21 333 296 de PROLABO
- Eau déminéralisée de qualité HPLC
- Sucrose octasulfate de potassium
- Acide orthophosphorique, par exemple, Carlo Erba code 406002 ou équivalent

Conditions chromatographiques

- HPLC waters alliance 2695
- Colonne NH$_2$
- Eluant : Solution aqueuse de sulfate d'ammonium tamponnée à pH = 3,00
- Débit : 1 mL / min
- Volume injecté : 50 $\mu$L
- Température de la colonne = 30°C
- Détection : réfractométrie (T int = 35°C)

Préparation des solutions étalons pour l'analyse chromatographique

On a réalisé une gamme d'étalonnage avec 3 témoins : 0,3 mg/mL - 1 mg/mL - 2,5 mg/mL

Le seuil de détection du sucrose octasulfate de potassium était de 0.06 mg/mL. Lorsqu'aucun pic n'a été détecté, le résultat a été majoré par le seuil de détection.

### Méthode de dosage de la sulfadiazine argentique

La sulfadiazine argentique a été dosée par Chromatographie Liquide Haute Performance (HPLC), dans les conditions suivantes :

Réactifs

- Eau de qualité HPLC
- Acide orthophosphorique, par exemple, Carlo Erba code 406002 ou qualité équivalente
- Acétonitrile
- Sulfadiazine argentique

Conditions chromatographiques

- Débit : 1 ml/min
- Température four colonne : 30°C
- Longueur d'onde $\lambda$ : 264 nm
- Phase éluante : eau / acétonitrile / acide orthophosphorique (Volumes respectifs : 900 / 99 / 1)

Préparation des solutions étalons pour l'analyse chromatographique

On a réalisé une gamme d'étalonnage avec 2 témoins : 0,4 mg/mL - 2,5 mg/mL.

Le seuil de détection de la sulfadiazine argentique était de 0,0006 mg/mL. Lorsqu'aucun pic n'a été détecté, le résultat a été majoré par le seuil de détection.

**Méthode de dosage : expression des résultats**

- On a tracé une droite d'étalonnage à l'aide des points d'étalonnage et on a calculé l'équation de la droite y = ax+b ($r^2 > 0,999$) dans laquelle y = surface sous le pic
  x = concentration de l'étalon (en mg/mL)
  $r^2$ = coefficient de détermination
- On a calculé la teneur en sucrose octasulfate de potassium ou sulfadiazine argentique (x).

Les résultats de l'HPLC ont été exprimés en mg/mL.

Le relargage de la substance active en pourcentage est calculé selon la formule suivante :

$$\text{Teneur}_\% = \frac{x \bullet V}{m \bullet T}$$

Dans laquelle :

Teneur% = relargage de la substance active par rapport à la teneur théorique dans le pansement.

X = relargage de substance active dans le sérum physiologique en mg/mL (donnée HPLC).

V : volume de sérum physiologique introduit lors de l'étude de relargage (10 mL ou 20mL pour les pansements absorbants)

m = masse de l'enduction (mg)

T = teneur en principe actif de l'enduction (%)

Les résultats des mesures de relargage ainsi obtenus sont mentionnés aux tableaux 4 à 7.

Tableau 4

| Relargage de l'actif dans les pansements interfaces des exemples 1 à 4 | | | | |
|---|---|---|---|---|
| | Exemple 1 | Exemple 2 | Exemple 3 | Exemple 4 |
| Relargage dela substance active (mg/mL) | 0,74 | 0,06 | 0,155 | 0,007 |

(suite)

| Relargage de l'actif dans les pansements interfaces des exemples 1 à 4 | | | | |
|---|---|---|---|---|
| | Exemple 1 | Exemple 2 | Exemple 3 | Exemple 4 |
| Relargage de la substance active (%) | 28,40 | 2,63 | 13,5 | 0,65 |

Tableau 5

| Relargage de l'actif dans des plaques de masses élastomériques | | | | | | | | |
|---|---|---|---|---|---|---|---|---|
| | Ex. 5 | Ex. 6 | Ex. 7 | Ex. 8 | Ex. 9 | Ex. 10 | Ex.11 | Ex. 12 |
| Relargage de la substance active (mg/mL) | 0,77 | 0,06 | 1,10 | 0,06 | 1,98 | 0,06 | 0,085 | 0,003 |
| Relargage de la substance active (%) | 15,38 | 1,21 | 19,56 | 1,14 | 10,07 | 0,31 | 0,91 | 0,03 |

Tableau 6

| Relargage de l'actif dans les pansements interfaces des exemples 13 à 18 | | | | | | |
|---|---|---|---|---|---|---|
| | Ex. 13 | Ex. 14 | Ex. 15 | Ex. 16 | Ex. 17 | Ex. 18 |
| Relargage de la substance active (mg/mL) | 3,01 | 1,16 | 6,02 | 0,32 | 0,393 | 0,098 |
| Relargage de la substance active (%) | 63,74 | 18,09 | 87,73 | 5,04 | 12,78 | 3,15 |

Tableau 7

| Relargage de l'actif dans les pansements interfaces complexés à des mousses des exemples 19 à 22 | | | | |
|---|---|---|---|---|
| | Ex. 19 | Ex. 20 | Ex. 21 | Ex. 22 |
| Relargage de la substance active (mg/mL) | 0,53 | 0,09 | 0,10 | 0,006 |
| Relargage de la substance active (%) | 50 | 9,89 | 17,97 | 1,02 |

Ces résultats montrent que bien que n'étant pas un tensioactif, comme le polysorbate 80 (Montanox 80, commercialisé par la société SEPPIC), un copolymère d'un sel de l'acide 2-méthyl-2-[(1-oxo-2-propènyl)amino]-1-propane-sulfonique et de l'ester 2-hydroxyéthyle de l'acide propénoïque permet un relargage de la substance active. Ces résultats montrent également que quelque soit la nature de la substance active (hydrosoluble ou non), le relargage a tout de même lieu.

Etude comparative

La capacité de relargage d'une substance active (telle que le sucrose octasulfate de potassium ou la sulfadiazine argentique) du copolymère précité a été mesurée dans des masses comprenant au moins un corps gras et comparée à celle obtenue avec un tensioactif, en l'occurrence le polysorbate 80 (Montanox 80 commercialisé par la société SEPPIC).

A cet effet, des masses élastomériques contenant du polysorbate 80 et une substance active (le sucrose octasulfate de potassium commercialisé par la société EUTICALS) ont été préparées selon le même procédé que pour les exemples 1 à 8, si ce n'est que le copolymère SEPINOV EMT 10 a été remplacé par du Montanox 80 commercialisé par la société SEPPIC.

Les quantités (exprimées en poids pour 100 grammes) des différents constituants des masses ainsi réalisées (exemples 23 et 24) sont indiquées dans le tableau 8.

Des pansements interfaces constitués d'une trame enrobée d'une masse élastomérique ont été élaborés à l'aide des masses ainsi réalisées.

Plus précisément, on a utilisé ici une trame formée d'une marquisette thermofixée en fils de polyester (polyéthylène téréphtalate), 33 décitex en chaîne et en trame, présentant des mailles carrées dont l'ouverture est d'environ 0,8 à 1 mm$^2$ (trame 555 commercialisée par la société MDB TEXINOV).

Cette trame a été enrobée d'une couche de masse fondue à 115°C, puis l'excédent a été éliminé par passage entre deux cylindres fixes dont l'écart est de 200 μm. La bande ainsi obtenue a été découpée puis complexée avec un film protecteur en polyester d'épaisseur de 23 μm sur chacune de ses faces formant ainsi des pansements individuels conditionnés dans des pochettes étanches et stérilisés sous rayonnement β à 25 kGy.

Tableau 8

| | Ondina 917 | Kraton G 1654 | Irgonix 1010 | Vaseline Codex | CMC Blanose A 7H4XF | Sucrose octasulfate de potassium | Sulfadiazine argentique | Montanox 80 |
|---|---|---|---|---|---|---|---|---|
| Exemple 23 | 62,38 | 6 | 0,12 | 5 | 14 | 7,5 | | 5 |
| Exemple 24 | 71,2 | 4,93 | 0,12 | 5 | 10 | | 3,75 | 5 |

Comparaison de la capacité de relargage d'une substance active entre le copolymère utilisé selon l'invention et un tensioactif de type polysorbate 80.

Les pansements des exemples 1 et 3 (contenant le copolymère Sepinov EMT 10 en tant qu'agent de relargage) et les pansements des exemples 23 et 24 (contenant le Montanox 80 en tant qu'agent de relargage) ont été testés selon la méthode de mesure du relargage d'une substance active de pansements interfaces décrite ci-dessus.

Les résultats obtenus sont présentés au tableau 9.

Tableau 9 : Relargage d'une substance active (exprimé en mg/mL et en pourcentage) à l'aide des masses selon les exemples 1, 23, 3 et 24

| | Exemple 1 | Exemple 23 | Exemple 3 | Exemple 24 |
|---|---|---|---|---|
| Relargage de la substance active (mg/mL) | 0,74 | 0,87 | 0,155 | 0,251 |
| Relargage de la substance active (%) | 28,4 | 32,94 | 13,5 | 19,6 |

Comme on peut le constater, on obtient sensiblement le même relargage de la substance active, quelle que soit sa nature, avec le Montanox® 80 ou le copolymère SEPINOV EMT 10.

**Mise en évidence de l'absence de cytotoxicité vis à vis des fibroblastes du copolymère utilisé dans le cadre de l'invention**

Pour mettre en évidence cette propriété avantageuse, des pansements interfaces ont été testés sur des cultures de fibroblastes selon la méthode suivante :

Matériels et méthodes

Cellules utilisées :

Type : pool de fibroblastes dermiques humains normaux (NHDF) R9PF2
Culture : 37°C, 5% CO2

Milieu de culture :

DMEM (Dulbecco's Modified Eagle Medium, Invitrogen 21969035). L-glutamine 2mM (Invitrogen 25030024) Pénicilline 50 UI/mL, streptomycine 50μg/mL (Invitrogen 15070063) Sérum de veau foetal 10% (v/v, Invitrogen 10270098)

Produits testés :

Des pansements selon les exemples 1 et 23 ont été découpés à la dimension des puits et testés.

Effets sur la prolifération :

Les fibroblastes ont été ensemencés à confluence en plaque 12 puits.

Des morceaux de chaque pansement ont été découpés à la taille 1,4 cm x 1,4 cm (soit 1,96 cm$^2$), appliqués à la surface des fibroblastes et maintenus à l'aide d'un bouchon. Un témoin sans pansement mais avec un bouchon, ainsi qu'un contrôle sans pansement et sans bouchon ont été réalisés.

Les cellules ont ensuite été incubées pendant 48 heures, 96 heures et 168 heures (7 jours) à 37°C et 5% de $CO_2$. Pour chaque temps d'incubation, l'activité métabolique a été mesurée par un test standard au MTT qui rend compte de l'activité des deshydrogénases mitochondriales. La thymidine tritiée ([méthyl-3H]-thymidine, Amersham TRK 686 2,5μCi/mL final) a été ajoutée pendant les 24 dernières heures d'incubation, puis l'ADN des cellules des tapis cellulaires a été extrait, purifié et la radioactivité incorporée dans l'ADN a été comptée à l'aide d'un compteur à scintillation.

Toutes les conditions ont été réalisées en triplicata. Les données brutes de comptage ont été transférées et traitées sous le logiciel PRISM® (Graph Pad Software).

Les résultats obtenus sont exprimés en coups par minute (cpm), puis en pourcentage par rapport au témoin selon la formule suivante :

$$\%_{\text{témoin}} = (\text{cpm}_{\text{test}} / \text{cpm}_{\text{témoin}}) \times 100$$

dans laquelle :

$\text{cpm}_{\text{test}}$ : nombre de coups par minute obtenus avec le test

$\text{cpm}_{\text{témoin}}$ : nombre de coups par minute obtenus avec le témoin

Les résultats obtenus figurent dans le tableau 10.

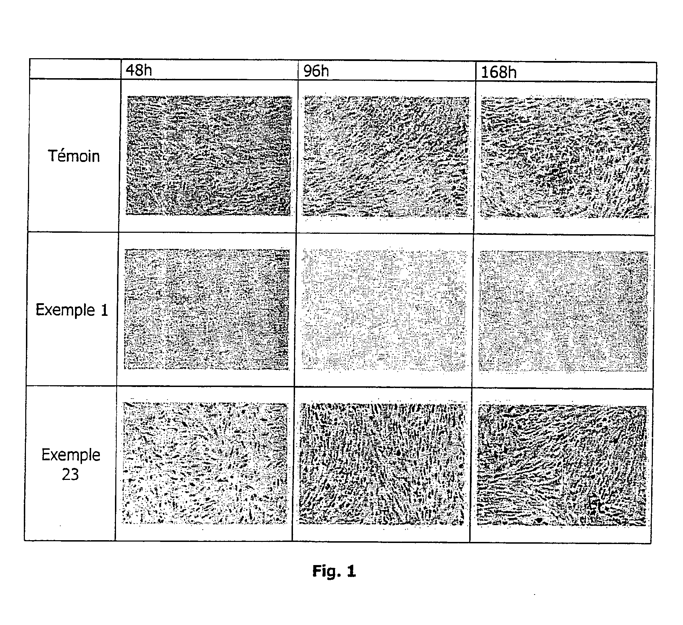

Des photographies des fibroblastes ont été réalisées à la fin du traitement après coloration au MTT. Ces photographies sont reproduites à la figure 1.

Tableau 10 : Viabilité des fibroblastes (exprimée en pourcentage) 48h, 96h, et 168h après traitement

| | Viabilité des fibroblastes à 48 heures (%) | Viabilité des fibroblastes à 96 heures (%) | Viabilité des fibroblastes à 168 heures (%) |
|---|---|---|---|
| Témoin | 100 | 100 | 100 |
| Contrôle sans bouchon | 98 | 103 | 107 |
| Pansement selon l'exemple 1 | 128 | 140 | 140 |
| Pansement selon l'exemple 23 | 70 | 68 | 57 |

Comme le montrent les résultats du tableau 10, l'utilisation du copolymère SEPINOV EMT 10 dans des pansements, dans des quantités équivalentes à un tensioactif tel que le Montanox 80, permet de relarguer une substance active dans des proportions équivalentes et présente un avantage supplémentaire qui est de favoriser la prolifération des fibroblastes.

La figure 1 montre l'effet des pansements des exemples 1 et 23 sur la viabilité cellulaire après contact direct sur les fibroblastes pendant 48h, 96h et 168h. Cette observation de la morphologie des fibroblastes après retrait des pansements et coloration au MTT a été visualisée par microscopie optique et prise de clichés représentatifs (objectif*10) et confirme l'effet bénéfique du copolymère SEPINOV EMT 10 sur la prolifération des fibroblastes par comparaison au Montanox 80.

## Revendications

1.  Utilisation, en tant qu'agent de relargage d'une substance active dans une composition pour pansement, d'un copolymère d'un sel de l'acide 2-méthyl-2-[(1-oxo-2-propènyl)amino]-1-propanesulfonique et de l'ester 2-hydroxyéthyle de l'acide propénoïque.

2.  Utilisation selon la revendication 1, **caractérisée en ce que** le sel de l'acide 2-méthyl-2-[(1-oxo-2-propènyl)amino]-1-propanesulfonique précité est un sel de sodium.

3.  Utilisation selon la revendication 1 ou 2, **caractérisée en ce que** la substance active précitée est choisie parmi les agents bactéricides ou bactériostatiques, les agents favorisant la cicatrisation, les enzymes favorisant la détersion de la plaie, les inhibiteurs de protéase ou de métalloprotéase, les agents antidouleurs, les anesthésiques locaux, les agents anti-inflammatoires non stéroïdiens.

4.  Utilisation selon l'une des revendications précédentes, **caractérisée en ce que** la composition comprend une matrice élastomérique.

5.  Utilisation selon l'une des revendications précédentes, **caractérisée en ce que** la composition comprend un hydrocolloïde.

6.  Pansement du type comprenant au moins un corps gras et/ou une matrice élastomérique et au moins une substance active, **caractérisé en ce qu'**il comprend un copolymère d'un sel de l'acide 2-méthyl-2-[(1-oxo-2-propènyl)amino]-1-propanesulfonique et de l'ester 2-hydroxyéthyle de l'acide propénoïque en tant qu'agent de relargage.

7.  Pansement selon la revendication 6, **caractérisé en ce qu'**il comprend de 0,1 à 20% en poids, de préférence de 1 à 10% en poids du copolymère précité.

8.  Pansement selon la revendication 6 ou 7, **caractérisé en ce qu'**il comprend de 0,01 à 15% en poids, de préférence de 3 à 8% en poids, de la substance active précitée.

9.  Pansement selon l'une des revendications 6 à 8, **caractérisé en ce que** le sel de l'acide 2-méthyl-2-[(1-oxo-2-propènyl)-amino]-1-propane-sulfonique est un sel de sodium.

10. Pansement selon l'une des revendications 6 à 9, **caractérisé en ce que** la substance active précitée est choisie parmi les agents bactéricides ou bactériostatiques, les agents favorisant la cicatrisation, les enzymes favorisant la détersion de la plaie, les inhibiteurs de protéase ou de métalloprotéase, les agents antidouleurs, les anesthésiques locaux, les agents anti-inflammatoires non stéroïdiens.

11. Pansement selon l'une des revendications 6 à 10, **caractérisé en ce qu'**il comprend un hydrocolloïde.

## Claims

1.  The use, as an agent for releasing an active substance in a composition for a dressing, of a copolymer of a salt of 2-methyl-2-[(1-oxo-2-propenyl)amino]-1-propanesulfonic acid and of 2-hydroxyethylpropenoate ester.

2.  The use as claimed in claim 1, **characterized in that** said salt of 2-methyl-2-[(1-oxo-2-propenyl)amino]-1-propanesulfonic acid is a sodium salt.

3.  The use as claimed in claim 1 or 2, **characterized in that** the abovementioned active substance is chosen from bactericidal or bacteriostatic agents, agents for promoting healing, enzymes for promoting wound cleaning, protease or metalloprotease inhibitors, painkillers, local anesthetics and nonsteroidal anti-inflammatory drugs.

4. The use as claimed in one of the preceding claims, **characterized in that** the composition comprises an elastomeric matrix.

5. The use as claimed in one of the preceding claims, **characterized in that** the composition comprises a hydrocolloid.

6. A dressing of the type comprising at least one fatty substance and/or one elastomeric matrix and at least one active substance, **characterized in that** it comprises a copolymer of a salt of 2-methyl-2-[(1-oxo-2-propenyl)amino]-1-propanesulfonic acid and of 2-hydroxyethylpropenoate ester, as a releasing agent.

7. The dressing as claimed in claim 6, **characterized in that** it comprises between 0.1% and 20% by weight, preferably between 1% and 10% by weight of said copolymer.

8. The dressing as claimed in claim 6 or 7, **characterized in that** it comprises between 0.01% and 15% by weight, preferably between 3% and 8% by weight, of the abovementioned active substance.

9. The dressing as claimed in one of claims 6 to 8, **characterized in that** the salt of 2-methyl-2-[(1-oxo-2-propenyl)amino]-1-propanesulfonic acid is a sodium salt.

10. The dressing as claimed in one of claims 6 to 9, **characterized in that** said active substance is chosen from bactericidal or bacteriostatic agents, agents for promoting healing, enzymes for promoting wound cleaning, protease or metalloprotease inhibitors, painkillers, local anesthetics and nonsteroidal anti-inflammatory drugs.

11. The dressing as claimed in one of claims 6 to 10, **characterized in that** it comprises a hydrocolloid.

**Patentansprüche**

1. Verwendung eines Copolymers eines Salzes der 2-Methyl-2-[(1-oxo-2-propenyl)amino]-1-propansulfonsäure und des 2-Hydroxyethylesters der Propensäure als Mittel zur Abgabe eines Wirkstoffes in einer Zusammensetzung für einen Verband.

2. Verwendung nach Anspruch 1, **dadurch gekennzeichnet, daß** das vorgenannte Salz der 2-Methyl-2-[(1-oxo-2-propenyl)amino]-1-propansulfonsäure ein Natriumsalz ist.

3. Verwendung nach Anspruch 1 oder 2, **dadurch gekennzeichnet, daß** der vorgenannte Wirkstoff aus den bakteriziden oder bakteriostatischen Substanzen, den wundheilungsfördernden Substanzen, den die Wundtoilette der Wunde begünstigenden Enzymen, den Protease- oder Metalloproteaseinhibitoren, den schmerzlindernden Substanzen, den Lokalanästhetika, den steroidfreien entzündungshemmenden Mitteln ausgewählt ist.

4. Verwendung nach einem der vorhergehenden Ansprüche, **dadurch gekennzeichnet, daß** die Zusammensetzung eine Elastomermatrix umfaßt.

5. Verwendung nach einem der vorhergehenden Ansprüche, **dadurch gekennzeichnet, daß** die Zusammensetzung ein Hydrokolloid umfaßt.

6. Verband vom Typ umfassend wenigstens ein Fett und/oder eine Elastomermatrix sowie wenigstens einen Wirkstoff, **dadurch gekennzeichnet, daß** er als Abgabemittel ein Copolymer eines Salzes der 2-Methyl-2-[(1-oxo-2-propenyl)amino]-1-propansulfonsäure und des 2-Hydroxyethylesters der Propensäure umfaßt.

7. Verband nach Anspruch 6, **dadurch gekennzeichnet, daß** er 0,1 bis 20 Gew.-%, vorzugsweise 1 bis 10 Gew.-% des vorgenannten Copolymers umfaßt.

8. Verband nach Anspruch 6 oder 7, **dadurch gekennzeichnet, daß** er 0,01 bis 15 Gew.-%, vorzugsweise 3 bis 8 Gew.-% des vorgenannten Wirkstoffes umfaßt.

9. Verband nach einem der Ansprüche 6 bis 8, **dadurch gekennzeichnet, daß** das Salz der 2-Methyl-2-[(1-oxo-2-propenyl)-amino]-1-propansulfonsäure ein Natriumsalz ist.

10. Verband nach einem der Ansprüche 6 bis 9, **dadurch gekennzeichnet, daß** der vorgenannte Wirkstoff aus den bakteriziden oder bakteriostatischen Substanzen, den wundheilungsfördernden Substanzen, den die Wundtoilette der Wunde begünstigenden Enzymen, den Protease- oder Metalloproteaseinhibitoren, den schmerzlindernden Substanzen, den Lokalanästhetika, den steroidfreien entzündungshemmenden Mitteln ausgewählt ist.

11. Verband nach einem der Ansprüche 6 bis 10, **dadurch gekennzeichnet, daß** er ein Hydrokolloid umfaßt.

|  | 48h | 96h | 168h |
|---|---|---|---|
| Témoin | | | |
| Exemple 1 | | | |
| Exemple 23 | | | |

**Fig. 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 272149 A
- EP 1272229 A
- WO 0170285 A
- FR 2392076
- FR 2495473
- WO 9810801 A
- EP 264299 A
- WO 0016723 A

**Littérature non-brevet citée dans la description**

- Wound dressings. Advances in Pressure Sensitive Adhesive Technology. Avril 1995, 158-171